(19) Europäisches Patentamt

European Patent Office

Office européen des brevets

(11) Veröffentlichungsnummer: **0 226 062**

**A1**

(12) # EUROPÄISCHE PATENTANMELDUNG

(21) Anmeldenummer: 86116071.1

(22) Anmeldetag: 20.11.86

(51) Int. Cl.⁴: **G01N 33/53** , G01N 33/574 , G01N 33/564 , G01N 33/577

(30) Priorität: 03.12.85 DE 3542677
23.08.86 DE 3628718

(43) Veröffentlichungstag der Anmeldung:
24.06.87 Patentblatt 87/26

(84) Benannte Vertragsstaaten:
AT BE CH DE ES FR GB IT LI NL SE

(71) Anmelder: **BAYER AG**
**Konzernverwaltung RP Patentabteilung**
**D-5090 Leverkusen 1 Bayerwerk(DE)**

(72) Erfinder: **Diamantstein, Tibor, Prof. Dr.**
**Platanen Allee 24**
**D-1000 Berlin 19(DE)**
Erfinder: **Osawa, Hisao, Dr.**
**Hans-Böhm-Zeile 30**
**D-1000 Berlin 37(DE)**
Erfinder: **Iosimovitc-Alasevic, Olivera,**
**Dipl.-Pharm.**
**Uhlandstrasse 67**
**D-1000 Berlin 31(DE)**

(54) Gelöste Interleukin-2-Rezeptoren als Indikator für Immunreaktionen und Neoplasien.

(57) Die Erfindung betrifft ein Verfahren zum Nachweis von Immunreaktionen, die im menschlichen oder tierischen Körper stattfinden, bei dem die in Körperflüssigkeiten gelöst vorliegenden Interleukin-2-Rezeptoren analytisch erfaßt werden. Weiterhin betrifft die Erfindung die Verwendung der gelöst vorliegenden Interleukin-2-Rezeptoren als Indikator für stattfindende Immunreaktionen und Neoplasien.

EP 0 226 062 A1

## Gelöste Interleukin-2-Rezeptoren als Indikator für Immunreaktionen und Neoplasien

Die Erfindung betrifft ein Verfahren zum Nachweis von Immunreaktionen, die im menschlichen oder tierischen Körper stattfinden, bei dem die in Körperflüssigkeiten gelöst vorliegenden Interleukin-2-Rezeptoren analytisch erfaßt werden. Weiterhin betrifft die Erfindung die Verwendung der gelöst vorliegenden Interleukin-2-Rezeptoren als Indikator für stattfindende Immunreaktionen und Neoplasien.

Das Interleukin-2 ist ein Wachstumsfaktor, der von Antigen-stimulierten T-Helfer-Lymphozyten synthetisiert und ausgeschieden wird. Damit das Interleukin-2 seine biologische Funktion, nämlich die Steigerung der Proliferation von bestimmten T-Lymphozyten, erfüllen kann, müssen diese Zielzellen einen spezifischen Rezeptor für das Interleukin-2 synthetisieren, der dann auf der Zellmembran erscheint.

Während andere von einem Wachstumsfaktor abhängige Zellen auf "ihren" Wachstumsfaktor praktisch immer ansprechen können, da sie die Rezeptormoleküle für "ihren" Faktor immer tragen, ist dies bei T-Lymphozyten nicht der Fall.

Solange sie ruhen, d.h. keinerlei Signal erhalten, tragen sie keinen Interleukin-2-Rezeptor und können auch in Gegenwart von Interleukin-2 nicht darauf mit Wachstum reagieren. In diesem Zustand der Ruhe befindet sich normalerweise der überwiegende Anteil der T-Lymphozyten. Wird das Immunsystem jedoch mit "fremd" konfrontiert, z. B. mit einem bestimmten Virusantigen, so kann ein kleiner Anteil von T-Lymphozyten durch seine spezifisch passenden Antigenrezeptoren dieses Virus erkennen. Nur diese T-Lymphozyten reagieren daraufhin mit der Bildung von Interleukin-2-Rezeptoren an ihrer Zelloberfläche und nur diese T-Lymphozyten werden wich in Gegenwart von Interleukin-2 und unter dessen Verbrauch vermehren.

Das Interleukin-2 wird an den auf der Zellmembran befindlichen Rezeptor gebunden und mit dem Rezeptor zuammen internalisiert. überraschenderweise wurde nun gefunden, daß bei einer Stimulation der T-Lymphozyten zum Beispiel durch ein Antigen nicht nur wie bis jetzt angenommen wurde Interleukin-2-Rezeptoren auf der Zellmembran von T-Lymphozyten erscheinen sondern auch in gelöstem Zustand in Körperflüssigkeiten wie zum Beispiel im Serum oder im Plasma auftreten. Warum die Rezeptoren auch in gelöstem Zustand auftreten, und welche Funktion sie in diesem Zustand haben ist zur Zeit noch unbekannt. Da die Existenz der zellgebundenen Interleukin-2-Rezeptoren wie oben beschrieben ein Marker für im menschlichen oder tierischen Körper stattfindende Immunreaktionen ist, ergibt sich nun überraschenderweise die Möglichkeit stattfindende Immunreaktion durch den Nachweis von in Körperflüssigkeiten gelöst vorliegenden Interleukin-2-Rezeptoren zu erkennen. Die Möglichkeit, die gelöst vorliegenden Rezeptoren nachzuweisen stellt eine wesentliche Vereinfachung der Analytik dar. Só ent fallen zum Beispiel arbeits-und geräteaufwendige Zellanreicherungen und Zellaufschlüsse, wie sie bisher für den Nachweis der auf der Zellmembran befindlichen Rezeptoren notwendig waren. Als Probenmaterial sind geeignet Körperflüssigkeiten, wie zum Beispiel Blut, Plasma, Serum, Liquor oder auch Lymphflüssigkeit.

Die Interleukin-2-Rezeptoren dienen als Indikator für stattfindende Immunreaktionen wie sie zum Beispiel bei Autoimmun-Erkrankungen (rheumatoide Arthritis, Morbus Bechterew, Diabetes, Multiple Sklerose usw.), Infektionen (Virusinfektionen, bakterielle Infektionen, Infektiöse Mononukleose usw.) und Allergien (Heuschnupfen, Arzneimittelallergien, Furunkulose, Asthma usw) auftreten. Weiterhin ermöglicht der Nachweis von gelöst vorliegenden Interleukin-2-Rezeptoren das Monitoring von Gewebeabstoßungsreaktionen im Zusammenhang mit Transplantationen (z.B. Nierentransplantationen), das Monitoring von Patienten bei der Interleukin-2-Therapie oder auch die Patientenüberwachung bei einigen Subtypen von Leukämie bzw. T-/B-Zell-Lymphomen (Mycosis Fungoides, Haar-Zell-Leukämie, Morbus Hodgkin usw.).

Nachgewiesen werden können die Rezeptoren mit den bekannten Methoden, die zum Nachweis von biospezifischen Rezeptoren geeignet sind oder auch mit immunologischen Nachweisverfahren wie zum Beispiel mit dem Radioimmuno-assay oder dem Enzyme-linked-immuno-assay.

Bedingt durch ihre hohe Empfinlichkeit und ausgeprägte Spezifität sind Nachweisverfahren mit Hilfe von Antikörpern als bevorzugt anzusehen, wobei die Verwendung von monoklonalen Antikörpern ganz besonders bevorzugt ist.

## Kulturmedium

Soweit nicht anders vermerkt, wurden die in dieser Anmeldung beschriebenen Zellinkubationen und Zellkultivierungen mit Serum-freiem Click's RMPI Medium (Seromed, D 8000 München) durchgeführt. Dieses Medium enthielt zusätzlich noch $2 \times 10^{-3}$ M L-Glutamin, $5 \times 10^{-5}$ M 2-Mercaptoethanol, 100 Einheiten/ml Penicillin und 100 µg/ml Streptomycin. Die Zellkultivierungen wurden bei 37°C durchgeführt.

## Zellen und Zell-Linien

Die im folgenden beschriebenen Zellen und Zellinien sind beispielhaft für Interleukin-2-Rezeptor positive bzw. negative Zellen. Mit Hilfe solcher Zellen wird gezeigt, daß Lymphozyten, die den Interleukinrezeptor an der Oberfläche exprimieren, diesen Rezeptor auch an die Umgebung abgeben. Für diesen Nachweis sind alle Interleukin-2-Rezeptor produzierenden Zellen geeignet.

## T-Lymphozyten:

Stimulierte T-Lymphozyten wurden gewonnen durch eine 3-tägige Inkubation von Milzzellen, die aus Balb/c Mäusen stammen, mit Concanavalin A. Dieses Pflanzenlektin ist in der Lage die Expression von Interleukin-2-Rezeptorprotein bei T-Helfer-Lymphozyten unspezifisch auszulösen. Nach dem Ende dieser Inkubation wurde das Concanavalin A unter Verwendung von α-Methyl-Mannosid von der Zelloberfläche entfernt. Einzelheiten dieser Prozedur sind von Osawa, H. und Diamantstein, T. in J. Immunol. 133 (1985), S. 1356 beschrieben.

## Eb und Esb Lymphom-Zellen:

Bei diesen Zellen handelt es sich um T-Lymphom-Zellen, die permanent Interleukin-2-Rezeptorprotein exprimieren. Diese Zell-Linien wurden aus DBA/2 Mäusen gewonnen. Einzelheiten über die Gewinnung und Eigenschaften der Zellen sind von Diamantstein, T. et al in British J. Cancer, 15 (1984) S. 23 beschrieben.

## EL-4 Zellen:

Diese Lymphomzell-Linie wurde aus C57B1/6 Mäusen gewonnen. Diese Zell-Linie exprimiert keinen Interleukin-2-Rezeptor. Einzelheiten über diese Zellinie sind in der oben genannten Literaturstelle von Diamantstein et al. beschrieben. Diese Zell-Linie dient als Negativkontrolle für die Experimente.

## Radioimmunoassay

Für den Test wurden zwei monoklonale Antikörper der Ratte gegen Maus Interleukin-2-Rezeptor verwendet. Der eine mit der Bezeichnung AMT13 ist ein Immunglobulin $G_{2a}$ (Osawa, H, und Diamantstein, T (1984) J. Immunol, 132, S. 2445) und der zweite mit der Bezeichnung 7D4 ist ein Immunglobulin M (Malek, T.R. et al (1983) Proc. Natl. Acad, Sci, USA, 80, S. 5694). Beide Antikörper erkennen unterschiedliche Epitope auf dem Interleukin-2-Rezeptor. Die Durchführung des Testes ist nicht von diesen monoklonalen Antikörpern abhängig. Es können grundsätzlich auch andere monoklonale oder polyklonale Antikörper verwendet werden, wie sie inzwischen auch kommerziell erhältlich sind.

Bei dem nachfolgend beschriebenen Test handelt es sich um einen sogenannten Sandwich-Radio-Immunoassay.

In flexible Miktrotiter-Platten (Titertec-PVC mit 96 Vertiefungen) wurden 100 µl von 7D4-Ascites-Flüssigkeit (1:1000 verdünnt mit Phosphat gepufferter Kochsalzlösung die 0,02% NaN₃ enthielt (PBS/NaN₃)) bei 4°C über Nacht inkubiert, um den Antikörper zu fixieren. Anschließend wurden die Platten noch mit PBS-NaN₃ welches 3% Rinderserumalbumin (PBS/BSA) enthielt bei 37°C inkubiert um die unspezifischen Bindungsstellen am PVC abzusättigen. Dann wurden die Platten zwei mal mit PBS das 0,1%Tween 20 - (PBS/Tween) enthielt gewaschen, anschließend jeweils 100 µl mit PBS verdünnte Probe (log 2-Verdünnung) in die Vertiefungen gegeben und für eine Stunde bei Raumtemperatur inkubiert.

3

Nach dreimaligem Waschen mit PBS/Tween wurden dann 100 µl [125] J-markierter AMT-13 verdünnt mit PBS/NaN$_3$ unter Zusatz von 0,5% BSA (4 x 10$^6$ cpm/ml) zugegeben und erneut für 1 Stunde bei Raumtemperatur inkubiert. Danach wurden die Vertiefungen noch dreimal mit PBS/Tween gewaschen, ausgeschnitten und mit einem Gammazähler ausgezählt.

Um unspezifische Bindungen zu erfassen, wurde als Kontrolle zum einen die Probe durch Verdünnungspuffer und in einer zweiten Kontrolle der Antikörper 7DA durch ein Immunglobulin M mit anderer Spezifität ersetzt.

Die unspezifische Bindung wurde substrahiert und die Zählraten gegen die Verdünnung der Proben aufgetragen.

Eine Einheit an Interleukin-2-Rezeptor ist definiert als die Menge (Proben-Verdünnung) die 50% der maximal möglichen Bindung ergibt.

Bestimmung der Rezeptorkonzentration auf den Zellen.

Die T-Lymphozyten und die Zellen der Zellinien Eb, Esb und E14 wurden unter Verwendung von Nonident® P40 in Anwesenheit von einem Protease-Inhibitor extrahiert (Osawa, H. und Diamantstein, T. - (1985) Europ, J. Immun., 15., S. 299). Dieser Extrakt wurde dann für 30 min bei 100,000 x g zentrifugiert und mit dem beschriebenen Radioimmunoassay analysiert. Die Ergebnisse sind in der Tabelle 1 aufgelistet.

Freisetzung von Interleukin-2-Rezeptor in vitro.

Mit diesem Experiment wurde geprüft ob die Interleukin-2-Rezeptor produzierenden Zellen diesen Rezeptor in einer in vitro-Kultur in das Kulturmedium abgeben. Dazu wurden die T-Lymphozyten, die Es, Eb und El-4 Zellen in dem oben beschriebenen Click's RPMI Medium für normalerweise 24 Stunden kultiviert. Den kulturen wurden die Überstände entnommen und zur Kontrolle die Lebendzellzahlen überprüft. Diese Überstände wurden durch Ultrafiltration 10 -20fach eingeengt und mit dem Radioimmunoassay analysiert. Die Resultate sind in der Tabelle 1 aufgelistet.

## Tabelle 1

Freisetzung von Interleukin-2-Rezeptor in vitro

| Experiment | Herkunft der Zellen | Einheiten an Interleukin-2-Rezeptor | |
|---|---|---|---|
| | | zellulär | freigesetzt |
| 1. | Eb | 210 | 83 |
| | Esb | 3 | 1 |
| | EL-4 | 0 | 0 |
| | Maus-T-Lymphoblasten | 120 | 52 |
| 2. | Eb | 72 | 77 |
| | EL-4 | 0 | 0 |
| | Maus-T-Lymphoblasten | 120 | 31 |
| 3. | Eb | 460 | 100 |
| | Maus-T-Lymphoblasten | 110 | 20 |

Freisetzung der Interleukin-2-Rezeptoren in vivo.

Mit diesem Experiment wurde geprüft, ob Interleukin-2-Rezeptor exprimierenden Zellen diesen Rezeptor auch in vivo abgeben. Dazu wurden $10^6$ Lymphomzellen (Esb, Eb und EL-4) intraperitoneal syngenen Mäusen (DBA/2 und C57B1/6) eingespritzt. Nach zwei Wochen wurde den Mäusen Blut entnommen und das Serum auf die Anwesenheit von Interleukin-2-Rezeptoren hin untersucht. Die Resultate, die in der Tabelle 2 aufgelistet sind, zeigen eindeutig, daß Zellen, die den Interleukin-2-Rezeptor exprimieren, diesen auch in vivo abgeben. Die Zellinie EL-4 ist eine Rezeptor-negative Zelle und diente als negativ-Kontrolle.

Tabelle 2:

Freisetzung von Interleukin-2-Rezeptor in vivo.

Serum-Probe

| Maus | | | Zellen | Einheiten an Interleukin-2-Rezeptor |
|---|---|---|---|---|
| DBA/2 | No. | 1. | Eb | 10 |
| | | 2. | Eb | 4 |
| | | 3. | Esb | 5 |
| | | 4. | Esb | 9 |
| C57B1/6 | No. | 1. | EL-4 | 0 |
| | | 2 | EL-4 | 0 |

ELISA für menschlichen IL-2-Rezeptor

Die Antikörper, AHT 107 (IgG₁) und AHT 54 (IgG₁) aus Ascites Flüssigkeit wurden durch aufeinanderfolgendes Binden und Eluieren an Protein-A-Sepharose (Deutsche Pharmazia GmbH, Munzinger Str. 9, D-7800 Frieburg 1) gemäß der Methode von Ey et al (Immunochemistry 15 (1978), 429) gereinigt.

In flexible Mikrotiter-Platten (Titerec-PVC mit 96 Vertiefunden -Flow Laboratories, 6701 Meckenheim) wurden 100 μl gereinigter AHT 107 (10 μl/ml PBS/NaN₃) gegeben und bei 4°C über Nacht inkubiert, um den Antikörper zu fixieren. Anschließend wurden die Platten noch mit PBS/NaN₃ welches 3 % Rinderserumalbumin (PBS/BSA) enthielt bei 37°C inkubiert um die unspezifischen Bindungsstellen am PVC abzusättigen. Dann wurden die Platten zwei mal mit PBS das 0,1 % Tween 20 (PBS/Tween) enthielt gewaschen. Anschließend werden jeweils 100 μl mit PBS verdünnte Serum-Proben (log 2-Verdünnung) in die Vertiefung gegeben.

Zur Kalibrierung wurde in parallelen gleichartigen Verdünnungen ein menschlicher IL-2-Rezeptor-Standard eingesetzt (Herstellung siehe unten). Die Verdünnung der Proben erfolgte mit PBS welches 1 % Triton X-100 und 2 mM Phenylmethansulfonylfluorid als Proteaseinhibitor enthielt (PBS/TX-100). Als Leerwert bei der Messung diente PBS/TX-100). Die Inkubation der Proben in den Mikrotiterplatten erfolgte für 1 Stunde bei 37°C. Nach dreimaligem Waschen mit PBS-Tween wurden dann 100 μl mit Biotin

markierter AHT 54 (0,5-1 μg/ml in PBS/NaN₃ enthaltend 1 % BSA) zugegeben und erneut für 1 Stunde bei 37°C inkubiert. Die Herstellung von Biotin markiertem AHT 54 wird unten beschrieben. Der Nachweis des gebundenen mit Biotin markierten AHT 54 erfolgte mit einem Streptavidin-Biotin-Peroxidase-Komplex - (SBPK, Amersham-Buchler, 3300 Braunschweig). Dazu wurden 100 μl einer 1:100 Verdünnung von SBPK in PBS enthaltend 1 % BSA zugegeben und für 1 Stunde bei 37°C inkubiert. Nach dreimaligem waschen mit PBS/Tween erfolgte der Peroxidasenachweis bei 37°C mit 100 μl einer Lösung von 0,55 mg/ml an 2,2-Azino-di-(3-ethylbenzthiazolinesulfonat) (ABTS®) in 0,1 M Citrat-/Phosphatpuffer, pH 5,3. Die Peroxidasereaktion wurde nach 30 Minuten durch Zugabe von 50 μl 0,1 M Zitronensäure enthaltend 0,01 % NaN₃ gestoppt und die Farbbildung bei 405 nm mit einem Titertek ELISA-Reader (Flow, Laboratories GmbH, 6701 Meckenheim) gemessen.

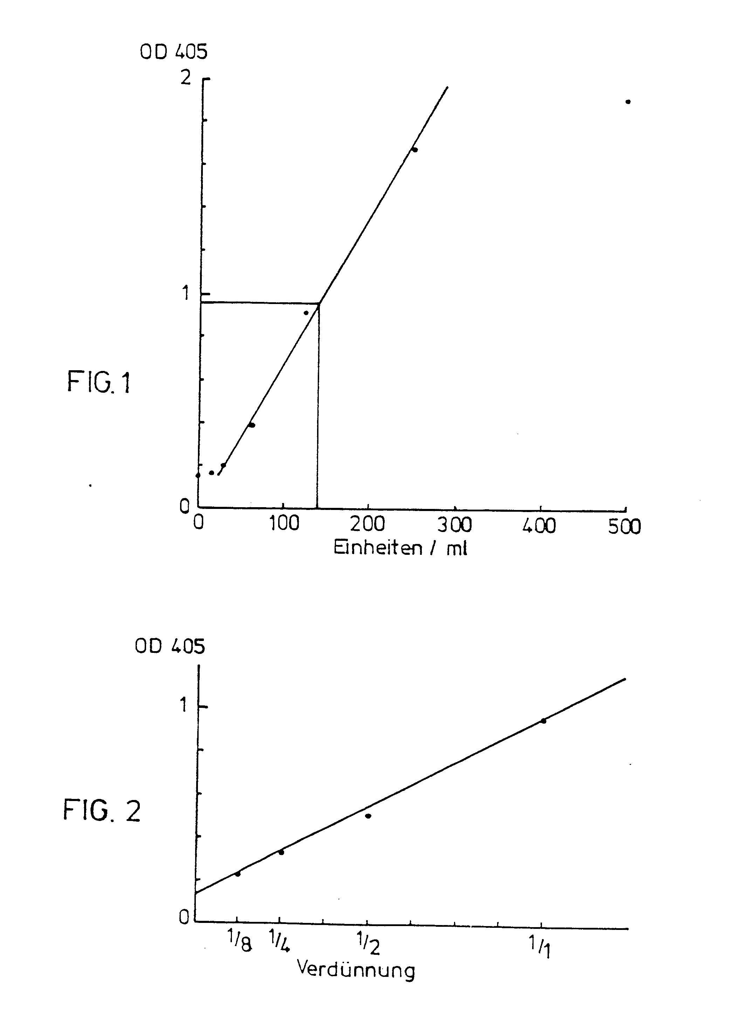

Die Fig. 1 zeigt die mit dem Standard erhaltene Kalibrationskurve. Aufgetragen ist Menge an Interleukin-2-Rezeptor in Einheiten/ml gegen die optische Dichte gemessen bei 405 nm. Die Fig. 2 zeigt die Resultate einer Verdünnungsreihe die mit einem Serum eines Patienten mit akuter T-Lyphozyten-Leukämie erhalten wurde. Aufgetragen ist hier die Verdünnung des eingesetzten Serums gegen die optische Dichte. Aus der bei dem unverdünnten Serum gemessenen $OD_{405}$ von 0,965 ergibt sich eine Menge von 142 Einheiten IL-2-Rezeptor/ml Serum. Bei als Vergleich mit getesteten Seren von gesunden Probanden konnte kein IL-2-Rezeptorprotein nachgewiesen werden. Der Test ist also spezifisch.

Herstellung von menschlichem IL-2-Rezeptor-Standard

Menschliche periphere Blut-Lymphozyten wurden 3 Tage mit 10 μg/ml Concanavalin A stimuliert - (Osawa, H. und Diamantstein, T., J. Immun. 133) und dann wurden die Zellen mit α-Methyl-Mannosid (20 mg/ml) behandelt und gewaschen. Anschließend wurden die Zellen in PBS/TX-100 lysiert (1 x 10$^8$ Zellen/ml) und das trübe Lysat zentrifugiert. Der klare Überstand wurde als IL-2-Rezeptor-Standard eingesetzt, wobei die Rezeptorkonzentration willkürlich auf 1000 Einheiten/ml festgelegt wurde.

Biotin-Markierung von AHT 54

0,2 ml des gereinigten AHT IgG (1 mg/ml in 0,1 M Borat-Puffer, pH 8,5) wurden mit 5 μl einer Lösung von 10 mg/ml Biotinyl-aminocapronsäure-N-hydroxy-succinimidester (Biotin-X-NHS; Calbiochem GmbH, D-6000 Frankfurt) in Dimethylsulfoxid gemischt. Dieses Mischung wurde 4 Stunden bei Raumtemperatur inkubiert und anschließend mit 40 μl 4 M Glycin und 10 μl Bromphenolblau (1 mg/ml) versetzt. Die Reinigung erfolgte über eine mit 12 mM Natriumphosphatpuffer, pH 7,4 enthaltend 0,15 M NaCl, 0,25 % Gelatine und 0,05 % NaN₃ äquilibrierte Sephadex G-50 Säule (1,5 ml).

Die IgG-Fraktionen die etwa 100 μg IgG/ml enthielten wurde gesammelt, durch einen Filter (Porengröße 0,22 μm) filtriert und bei 4°C gelagert.

**Ansprüche**

1) Verfahren zum Nachweis von im menschlichen oder tierischen Körper stattfindenen Immunreaktionen und von Neoplasien, dadurch gekennzeichnet, daß man die in Körperflüssigkeiten gelöst vorliegenden Interleukin-2-Rezeptoren analytisch erfaßt.

2) Verfahren nach Anspruch 1, dadurch gekennzeichnet, daß man die in Körperflüssigkeiten gelöst vorliegenden Interleukin-2-Rezeptoren mit Hilfe von Antikörpern nachweist, die gegen die Interleukin-2-Rezeptor gerichtet sind.

3) Verfahren nach den Ansprüchen 1 und 2, dadurch gekennzeichnet, daß der Nachweis der Interleukin-2-Rezeptoren mit einem Radioimmunoassay, Enzyme-linked-immunoassay oder einem Fluoreszenzimmunoassay erfolgt.

4) Verfahren nach den Ansprüchen 1 bis 3 zum Nachweis von im Blut, Serum, Plasma, Liquor oder Lymphflüssigkeit gelöst vorliegenden Interleukin-2-Rezeptoren.

5) Verfahren nach den Ansprüchen 1 bis 4 zum erkennen von Immunreaktionen die durch Autoimmun-Erkrankungen, Allergien, Gewebe-Abstoßungsreaktionen oder Infektionen ausgelöst wurden.

6) Verfahren nach den Ansprüchen 1 bis 4 zum Erkennen und Überwachen von Subtypen von Leukämien bzw. von T/B-Zell-Lymphomen.

7) Verwendung von in Körperflüssigkeiten gelöst vorliegenden Interleukin-2-Rezeptoren als Indikator für im menschlichen oder tierischen Körper stattfindende Immunreaktionen.

8) Verwendung von in Körperflüssigkeiten gelöst vorliegenden Interleukin-2-Rezeptoren nach Anspruch 7 zum Erkennen von Immunreaktionen bei Autoimmun-Erkrankungen, Allergien, Infektionen und Gewebe-Abstoßungsreaktionen.

9) Verwendung von in Körperflüssigkeiten gelöst vorliegenden Interleukin-2-Rezeptoren zum Erkennen und Überwachen von Subtypen von Leukämien und T/B-Zell-Lymphomen.

FIG. 1

FIG. 2

Europäisches Patentamt

# EUROPÄISCHER RECHERCHENBERICHT

Nummer der Anmeldung

EP 86 11 6071

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | THE JOURNAL OF IMMUNOLOGY, Band 135, Nr. 5, November 1985, Seiten 3172-3177, The American Association of Immunologists, US; L.A. RUBIN et al.: "Soluble interleukin 2 receptors are released from activated human lymphoid cells in vitro" * Insgesamt * | 1-9 | G 01 N 33/53 G 01 N 33/574 G 01 N 33/564// G 01 N 33/577 |
| | --- | | |
| Y | THE JOURNAL OF IMMUNOLOGY, Band 134, Nr. 4, April 1985, Seiten 2387-2392, The American Association of Immunologists, US; A.W. BOYD et al.: "Structural and functional characterization of IL 2 receptors on activated human B cells" * Insgesamt * | 1-9 | |
| | --- | | RECHERCHIERTE SACHGEBIETE (Int. Cl.4) |
| Y | THE JOURNAL OF IMMUNOLOGY, Band 134, Nr. 4, April 1985, Seiten 2393-2404, The American Association of Immunologists, US; R. MITTLER et al.: "Activated human B cells display a functional IL 2 receptor" * Insgesamt * | 1-9 | G 01 N C 12 P C 12 N A 61 K |
| | ---                    -/- | | |

Der vorliegende Recherchenbericht wurde für alle Patentansprüche erstellt.

| Recherchenort | Abschlußdatum der Recherche | Prüfer |
|---|---|---|
| DEN HAAG | 19-03-1987 | OSBORNE H.H. |

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | THE JOURNAL OF IMMUNOLOGY, Band 134, Nr. 4, April 1985, Seiten 2405-2413, The American Association of Immunologists, US; T.R. MALEK et al.: "The murine IL 2 receptor" * Insgesamt * | 1-9 | |
| Y | PROCEEDINGS OF THE NATIONAL ACADEMY OF SCIENCES OF USA, Band 82, Februar 1985, Seiten 864-868; K.A. SMITH et al.: "Interleukin 2 regulates its own receptors" * Insgesamt * | 1-9 | |
| Y | EP-A-0 111 344 (SLOAN-KETTERING INSTITUTE FOR CANCER RESEARCH) * Zusammenfassung; Vorrede; Ansprüche 13-16 * | 1-9 | RECHERCHIERTE SACHGEBIETE (Int. Cl 4) |
| A | EP-A-0 162 699 (IMMUNEX CORP.) * Zusammenfassung; Seiten 1-9 * | 1 | |

--- -/-

Der vorliegende Recherchenbericht wurde für alle Patentansprüche erstellt.

| Recherchenort | Abschlußdatum der Recherche | Prüfer |
|---|---|---|
| DEN HAAG | 19-03-1987 | OSBORNE H.H. |

Europäisches
Patentamt

**EUROPÄISCHER RECHERCHENBERICHT**

Nummer der Anmeldung

EP 86 11 6071

Seite 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | CHEMICAL ABSTRACTS, Band 104, Nr. 21, 26. Mai 1986, Seite 470, Zusammenfassung Nr. 184414h, Columbus, Ohio, US; T. UCHIYAMA et al.: "Abnormal expression of interleukin-2 receptor (Tac antigen) in adult T-cell leukemia", & PROC. INT. SYMP. PRINCESS TAKAMATSU CANCER RES. FUND 1984, (Pub. 1985), 15th(Retroviruses Hum. Lymphoma/Leuk.), 253-8 * Insgesamt * | 1 | |
| T | THE JOURNAL OF IMMUNOLOGY, Band 138, Nr. 1, 1. Januar 1987, Seiten 192-196, The American Association of Immunologists, US; A.B. RESKE-KUNZ et al.: "Soluble interleukin 2 receptors are released by long term-cultured insulin-specific T cells transiently after contact with antigen" | 1 | RECHERCHIERTE SACHGEBIETE (Int. Cl 4) |

Der vorliegende Recherchenbericht wurde für alle Patentansprüche erstellt.

| Recherchenort | Abschlußdatum der Recherche | Prüfer |
|---|---|---|
| DEN HAAG | 19-03-1987 | OSBORNE H.H. |